(19) Europäisches Patentamt
European Patent Office
Office européen des brevets

(11) EP 4 043 085 A1

(12) EUROPEAN PATENT APPLICATION
published in accordance with Art. 153(4) EPC

(43) Date of publication:
**17.08.2022 Bulletin 2022/33**

(21) Application number: **20873989.6**

(22) Date of filing: **09.10.2020**

(51) International Patent Classification (IPC):
***B01D 39/16*** *(2006.01)* ***A61M 1/02*** *(2006.01)*

(52) Cooperative Patent Classification (CPC):
**A61M 1/02; B01D 39/16**

(86) International application number:
**PCT/JP2020/038276**

(87) International publication number:
**WO 2021/070928 (15.04.2021 Gazette 2021/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.10.2019 JP 2019187513**

(71) Applicant: **Asahi Kasei Medical Co., Ltd.**
**Tokyo 100-0006 (JP)**

(72) Inventor: **SHIMADA, Nobukazu**
**Tokyo 100-0006 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) BLOOD TREATMENT FILTER AND METHOD FOR MANUFACTURING SAME, AND LEUKOCYTE REMOVAL METHOD

(57) An object is to provide a blood processing filter favorable in both of the effectiveness (leukocyte removing performance) and the safety (reduction in the amount of elutable substances). The object can be achieved by a blood processing filter comprising a filtration medium including a polyester fiber, wherein a surface area of the filtration medium is 6.0 $m^2$ or more, and a maximum absorbance of an aqueous extract of the blood processing filter in the range from 240 to 245 nm is 0.03 or less.

[Fig. 4]

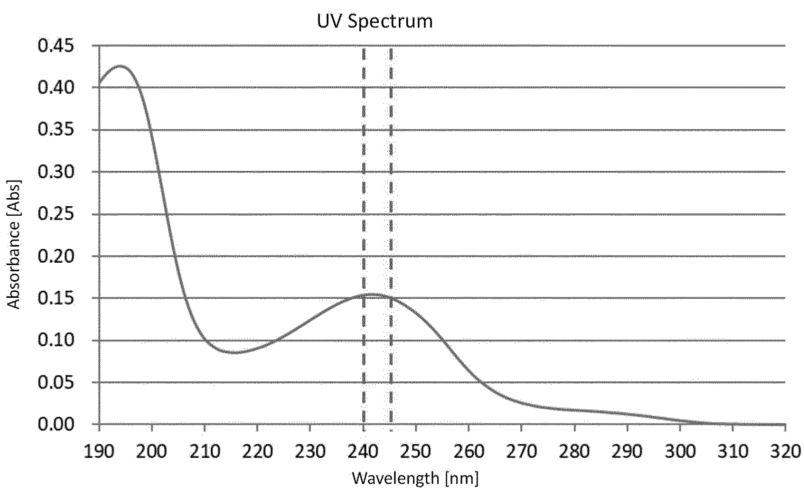


EP 4 043 085 A1

## Description

### Technical Field

The present invention relates to a blood processing filter, a method for producing the same, and a method for removing leukocyte. The present invention preferably relates to a blood processing filter for removing undesirable components such as aggregates and leukocytes from blood, that is, a liquid containing blood components, such as whole blood and blood preparations (liquids prepared and obtained from whole blood, and liquids obtained by adding various additives thereto), a method for producing the same, and a method for removing leukocyte involving use of the blood processing filter.

### Background Art

In the field of blood transfusion, so-called the component transfusion, which includes separating a blood component needed by a blood recipient from a whole blood preparation and infusing the blood component, has been commonly carried out, in addition to so-called the whole blood transfusion, which includes transfusing a whole blood preparation obtained by adding an anticoagulant agent to blood collected from a blood donor. The component transfusion includes erythrocyte transfusion, thrombocyte transfusion, and blood plasma transfusion, depending on the type of blood component needed by a blood recipient, and the blood preparations used for these transfusions include erythrocyte preparations, thrombocyte preparations, and blood plasma preparations.

Additionally, so-called the leukapheresis blood transfusion, which includes removing leukocytes included in a blood preparation and then transfusing the resulting blood preparation, has been widely used in recent years. This is because it is now clear that comparatively mild side effects associated with blood transfusion, such as headache, nausea, chill, and nonhemolytic febrile reaction, and severe side effects, such as alloantigen sensitization, virus infection, and post-transfusion GVHD, which result in grave consequences to a blood recipient are predominantly caused by the leukocytes mixed in a blood preparation used for blood transfusion. It is said that, for preventing comparatively mild side effects, such as headache, nausea, chill, and fever, leukocytes in a blood preparation may be removed to a residual rate of $10^{-1}$ to $10^{-2}$ or less. It is said that, for preventing the side effects such as HLA antibody production and post-transfusion GVHD, leukocytes in a blood preparation may be removed to a residual rate of $10^{-3}$ or less. It is said that, for preventing the severe side effects such as alloantigen sensitization and virus infection, leukocytes need to be removed to a residual rate of $10^{-4}$ to $10^{-6}$ or less.

Further, leukapheresis by extracorporeal blood circulation has been practiced in recent years for treating diseases such as rheumatism and ulcerative colitis, and high clinical effects have been exhibited.

Currently, there are roughly two kinds of the methods for removing leukocytes from a blood preparation: centrifugation methods including separating and removing leukocytes utilizing specific gravity differences of blood components using a centrifuge; and filter methods including removing leukocytes using a filter element made of fiber assemblies such as nonwoven fabrics or a porous material having continuous pores. Filter methods for removing leukocytes by adhesion or adsorption are the most prevalent at present due to advantages such as a simple operation and a low cost.

Design and improvement of filter elements used for the above filter methods have been advanced, and a wide variety of inexpensive filters with high adsorption performance on leukocytes are sold by various manufacturers. On the other hand, safety requirements for leukocyte removing filters in accordance with the guidelines of ISO (International Organization for Standardization) are increasingly stringent in recent years.

Conventionally, a biological safety test on a leukocyte removing filter defined by ISO 10993 has been essential for the administrative procedures such as a pharmaceutical application. However, the conformity is also increasingly demanded by medical institutions of various countries to filter manufactures, in terms of chemical safety of a single-time use blood transfusion set including a leukocyte removing filter defined by ISO 1135-4 (Transfusion equipment for medical use), and chemical safety of filter composition members in accordance with Pharmacopoeia of various countries defined by ISO 3826-3, for example.

Further, separately from the ISO standards, China, for example, establishes its own standard YY0329 for single-time use leukocyte removing filters and strictly defines the chemical and biological safeties in addition to the filtration performance.

Patent Literature 1 is a literature with a focus on the chemical elution of a leukocyte removing filter. Patent Literature 1 discloses that an oligomer content is decreased by washing treatment of a filter substrate installed in a leukocyte removing filter to enhance the leukocyte removing performance.

In addition, the invention disclosed in Patent Literature 2 is an invention with a focus on the surface properties of fibers for a blood filter. This intends to enhance performances by optimizing an amount of the terminal carboxyl group included in the fiber. Here, the terminal carboxyl group is a functional group included in a fiber-derived oligomer, and the optimization of terminal carboxyl group accordingly suggests the control on the oligomer in the fiber.

Citation List

Patent Literature

Patent Literature 1: Japanese Patent No. 4565762
Patent Literature 2: International Publication No. WO2018/034213
Patent Literature 3: Japanese Patent Laid-Open No. 2006/077136

Summary of Invention

Technical Problem

Here, the present inventors have carried out the safety confirmation in accordance with the above ISO standards on leukocyte removing filters having fibers as a filtration material and distributed in various countries, and have surprisingly found that all of the filters did not conform with the chemical safety in accordance with ISO 1135-4. Specifically, the filters have failed in either the residual amount on evaporation (5 mg or less) in an aqueous extract defined by "6.4 Residue on evaporation" of ISO 1135-4, or the absorbance (0.1 or less) of an aqueous extract defined by "6.5 UV absorption of extract solution".

The above results show that the fibers filled in various filters include a water-soluble component at least on the surface, or include a water-insoluble and physically unstable substance, which peels and falls off when water is in contact with the fiber.

As already have been mentioned, ISO 1135-4 is the standard required on single-time use blood transfusion sets and applicable to, for example, a transfusion set when blood is transfused to a patient at the bedside in a medical facility such as a hospital. Thus, such a standard is not applicable to bag sets with a leukocyte removing filter for in-line use, which are currently the mainstream in advanced countries. Despite lack of conformity with ISO 1135-4, these filters are not illegal in view of the purpose of use at the moment.

Various filters are inevitably conformable with the biological safety as the ISO 10993 leukocyte removing filters, and thus results of the ISO 1135-4 test do not directly suggest the harmfulness or toxicity of the filters but cannot be underestimated from a long-term viewpoint when considering, for example, impacts on health conditions of frequently blood transfused patients.

It is notable that, for decreasing the components eluted from fiber materials to conform with ISO 1135-4, it is primarily preferable to decrease the amount of the fiber material used for a filter or to increase the average fiber diameter of the fibers composing a fiber material. However, the leukocyte removing function will be lost when the amount of the fiber material is decreased or when the diameter of the fiber material is increased. When the leukocyte removing function reduces, the risk of side effects of blood transfusion caused by leukocytes is increased, and hence unacceptable for safety reason.

The above Patent Literature 3 reports an example with a focus on the actual decrease of eluted components, and specifically reports that the maximum absorbance in a wavelength range from 220 to 350 nm can be decreased to about 0.003 by purifying a coating polymer to be applied to a leukocyte removing filter material thereby to remove low-molecular components.

However, the leukocyte removing rate disclosed in Example 4 of Patent Literature 3 is as significantly low as 80.5%, which is not the performance acceptable for a blood transfusion preparation. For enhancing the leukocyte removing function of the nonwoven fabric of Patent Literature 3, the amount of the nonwoven fabric to be used must be increased or the average fiber diameter of the fibers composing the nonwoven fabric must be decreased. As described hereinbefore, either means is difficult to practically implement because there is a high risk that eluted components derived from a fiber material may increase significantly.

The present inventors have confirmed that there is a relative relationship between the specific surface area of a nonwoven fabric and the maximum absorbance of a nonwoven fabric extract solution. Specifically, when a polybutylene terephthalate nonwoven fabric is used, the specific surface area reduces as the average fiber diameter thereof increases, and consequently the liquid contact area reduces, thereby reducing the absorbance.

It is specially notable that the specific surface area and the absorbance are in the proportional relationship substantially through the origin. It is commonly known that the leukocyte removing function depends on the surface area of a fiber material in a filter, but the absorbance is simultaneously in the direct proportional relationship with the surface area, thereby making the absorbance and the leukocyte removing function be in the direct proportional relationship. It is thus understood that the simple controls on the amount of the fiber material and the fiber diameter are unable to achieve the compatibility between the reduction in the absorbance and the enhancement of the leukocyte removing

function.

However, in the prediction of expansion of the leukocyte removing filter market from now on, the desire lies in the realization of a filter which is inexpensive and also enhances the safety of the blood transfusion preparations more than conventional filters and can be used reliably in view of both the decrease in the side effects of blood transfusion and the decrease in elutable substances.

Thus, the present invention aims to provide a blood processing filter favorable in both of the effectiveness (leukocyte removing performance) and the safety (reduction in the amount of elutable substances).

Solution to Problem

The present inventor have carried out again the qualitative analysis by the LC/MC method on the aqueous extracts of the filters that were not conformable with ISO 1135-4 and consequently identified a plurality of monomer components and oligomer components having the terephthalic acid skeleton. Examples of the monomer component and the oligomer component may include the following components.

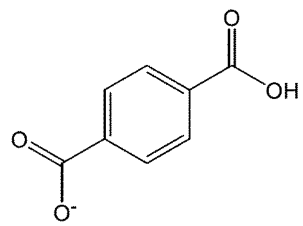

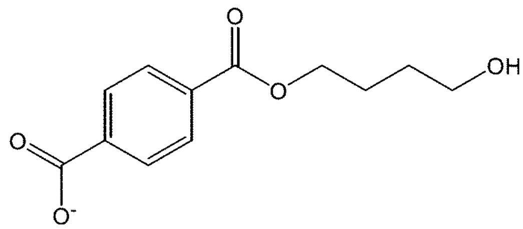

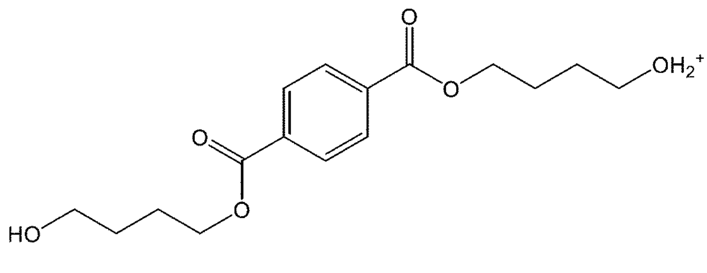

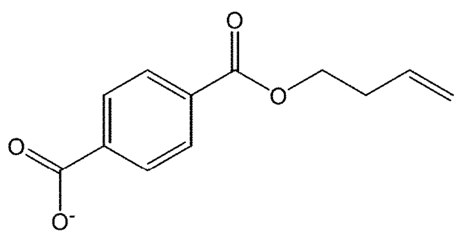

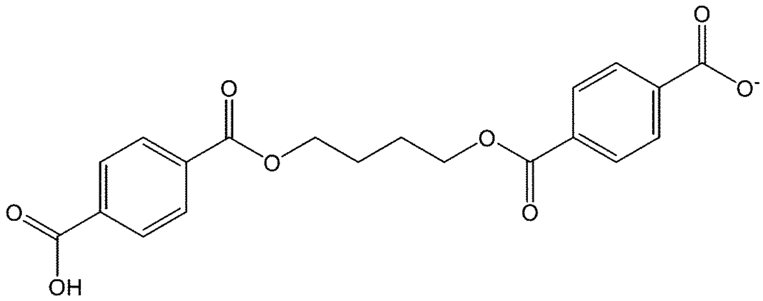

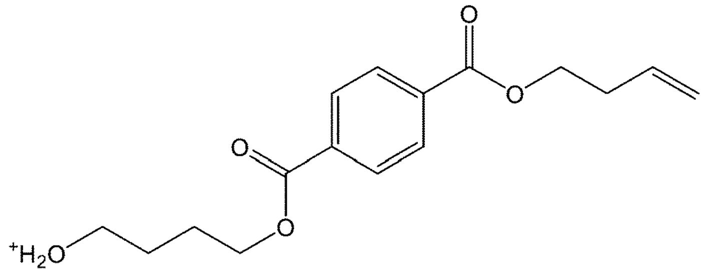

For example, in the case of the filters having a fiber including polybutylene terephthalate as the main component, terephthalic acid and butyl terephthalate were detected in the extract solution. The mechanisms of the generation thereof are unclear but it is presumed that they probably remain as unreacted components during a polymerization of a polycondensation polymer, or generate later through the pyrolysis during the fiber spinning or the hydrolysis during sterilization treatment of a product.

The present inventors have conducted extensive studies and consequently have found that the amounts of the monomer components and the oligomer components included in blood processing filters can be decreased by employing various methods (specifically, washing, low-temperature spinning, or vacuum venting). Since the amounts of the monomer components and the oligomer components included in blood processing filters are extremely small, the quantification thereof is difficult. The present inventors have focused on the intense absorptions of the monomer components and the oligomer components in an aqueous solution in the range from 240 to 245 nm (for example, Figure 4) and also found that the monomer components and the oligomer components can be alternatively quantified by measuring the absorbances in this wavelength range (specifically, the amounts of the monomer components and the oligomer components can be expressed in terms of the absorbance in the range from 240 to 245 nm).

Patent Literature 3 discloses a technology for decreasing water-eluting components of a polymer by purifying a coating polymer material, and then the present inventors have examined the absorbance spectra of components derived from a polyethylene terephthalate nonwoven fabric, which is a common fiber material, and a copolymer of 2-hydroxyethyl (meth)acrylate/dimethylaminoethyl methacrylate in 97:3, which is a common hydrophilic polymer, in the UV wavelength range. As a result, it has been verified that the absorbance in the range from 250 to 320 nm is predominant by the oligomer components derived from the fiber material, and that the absorbances of the components derived from the hydrophilic polymer were shifted to shorter wavelength ranges. Accordingly, the amount of impurities derived from the fiber material cannot be decreased even when the design and amount of hydrophilic polymer applied are modified.

Specifically, the present invention is as follows.

[1] A blood processing filter comprising:

a filtration medium including a polyester fiber,
wherein a surface area of the filtration medium is 6.0 $m^2$ or more, and
a maximum absorbance of an aqueous extract of the blood processing filter in the range from 240 to 245 nm is 0.03 or less.

[2] The blood processing filter according to [1], wherein a pressure loss of the blood processing filter in ventilation is 100 to 2000 Pa.
[3] The blood processing filter according to [1] or [2], wherein a pressure loss of the blood processing filter in ventilation is 400 to 1600 Pa.
[4] The blood processing filter according to any of [1] to [3],
wherein, when 400 mL of a leukocyte-containing liquid having a leukocyte concentration of 5000 cells/μL is filtered,

a leukocyte removing performance is 3.3 or more, wherein the leukocyte removing performance is represented by a formula below:

leukocyte removing performance = −log [(leukocyte concentration in leukocyte-containing liquid after filtration)/(leukocyte concentration in leukocyte-containing liquid before filtration)].

[5] The blood processing filter according to any of [1] to [3], wherein when 400 mL of a leukocyte-containing liquid having a leukocyte concentration of 5000 cells/μL is filtered, a residual white blood cell count is less than $10^6$ cells.
[6] The blood processing filter according to any of [1] to [5], wherein a surface area of the filtration medium is 10.0 $m^2$ or less.
[7] The blood processing filter according to any of [1] to [6], wherein a surface area of the filtration medium is 8.5 $m^2$ or less.
[8] The blood processing filter according to any of [1] to [7], wherein the maximum absorbance per unit mass (g) of the filtration medium is 0.003 $g^{-1}$ or less.
[9] The blood processing filter according to any of [1] to [8],

wherein the polyester fiber has a coating layer,
a mass of the coating layer per total mass (g) of the polyester fiber and the coating layer is 15 mg/g or less.

[10] A method for removing leukocyte, comprising the step of passing a leukocyte-containing liquid through the blood processing filter according to any of [1] to [9].
[11] The method for removing leukocyte according to [10] wherein a leukocyte residual rate by the passing is $10^{-3}$ or less, wherein the leukocyte residual rate is represented by a formula below:

leukocyte residual rate = (leukocyte concentration in leukocyte-containing liquid after filtration)/(leukocyte concentration in leukocyte-containing liquid before filtration).

[12] The method for removing leukocyte according to [10], wherein a residual white blood cell count in 400 mL of a leukocyte-containing liquid by the passing is less than $10^6$ cells.
[13] A method for producing a blood processing filter comprising a filtration medium including a polyester fiber, comprising:

the step of washing the polyester fiber with an ethanol aqueous solution,
wherein a concentration of the ethanol aqueous solution is 12% (v/v) or more, and
a temperature of the ethanol aqueous solution is 20°C or more.

[14] A method for producing a blood processing filter comprising a filtration medium including a polyester fiber, comprising:
the step of spinning the polyester fiber at 260°C or less.
[15] A method for producing a blood processing filter comprising a filtration medium including a polyester fiber, comprising:
the step of vacuum venting a polyester source of the polyester fiber.

Advantageous Effects of Invention

According to the present invention, a blood processing filter favorable in both of the effectiveness (leukocyte

removing performance) and the safety (reduction in the amount of elutable substances) can be provided.

Brief Description of Drawings

[Figure 1] Figure 1 is a schematic diagram of the blood processing filter of an embodiment of the present invention.
[Figure 2] Figure 2 is a cross-section drawing of the blood processing filter shown in Figure 1.
[Figure 3] Figure 3 shows an apparatus for preparing an aqueous extract of a blood processing filter.
[Figure 4] Figure 4 is a figure showing that monomer components and oligomer components exhibit high absorbances in the range from 240 to 245 nm.

Description of Embodiments

Hereinafter, embodiments to carry out the present invention (hereinafter, referred to as "the present embodiment") will be described in detail. The present invention is not limited to the following embodiments and can be carried out in various modifications within the scope of the gist.
Hereinafter, the term "blood" encompasses blood and blood component-containing liquids unless otherwise specified. Examples of the blood component-containing liquid include blood preparations. Examples of the blood preparation include whole blood preparations, erythrocyte preparations, thrombocyte preparations, and blood plasma preparations.

<Blood processing filter>

One of the present embodiments relates to a blood processing filter comprising a filtration medium including a polyester fiber,

wherein a surface area of the filtration medium is 6.0 $m^2$ or more, and
a maximum absorbance of an aqueous extract of the blood processing filter in the range from 240 to 245 nm is 0.03 or less.

Both of the effectiveness (leukocyte removing performance) and the safety (reduction in the amount of elutable substances) can be achieved when the blood processing filter including filtration medium with a certain surface area or more gives an aqueous extract exhibiting a maximum absorbance of 0.03 or less in the range from 240 to 245 nm while (amounts of the monomer components and the oligomer components are decreased).
The blood processing filter of the present embodiment includes, for example, a container having an inlet and an outlet for blood, and a filtration medium disposed between the inlet and the outlet.
For example, the blood processing filter of the present embodiment can be configured to include a filtration medium and a container component on the inlet side and another container component on the outlet side disposed so as to interpose the filtration medium, wherein the container component on the inlet side and the container component on the outlet side have grip parts for gripping the outer-edge part of the filtration medium by grasping.
Figure 1 is a schematic diagram of an embodiment of such a blood processing filter (leukocyte removing filter), and Figure 2 is a cross-sectional view along line II-II of Figure 1.
As shown in Figure 1 and Figure 2, blood processing filter 10 has a flat container 1 and a filtration medium 5, which is contained therein and substantially in a dry state. The container 1 containing the filtration medium 5 includes two elements, a container component on the inlet side having an inlet 3 and another container component on the outlet side having an outlet 4. The filtration medium 5 divides the space in the flat container 1 into a space on the inlet side 7 and a space on the outlet side 8.
This blood processing filter 10 has a structure in which the container component on the inlet side and the container component on the outlet side are disposed so as to interpose the filtration medium 5, wherein the two container components grip the outer-edge part 9 of the filtration medium 5 by grasping with grip parts provided at a part thereof.
In another embodiment, the filtration medium and the containers may be joined by welding and the like, thereby to give a structure in which the filtration medium is gripped by the containers.

[Container]

Examples of the material for the container include rigid resins and flexible resins.
Examples of the rigid resin include phenol resins, acrylic resins, epoxy resins, formaldehyde resins, urea resins,

silicon resins, ABS resins, nylon, polyurethanes, polycarbonates, vinyl chloride, polyethylene, polypropylene, polyester, and styrene-butadiene copolymers, with polycarbonates being preferable due to good thermal stability and formability.

The flexible resin is preferably those having the thermal and electrical properties similar to those of the filtration medium. Examples of the flexible resin include polyolefins such as soft polyvinyl chloride, polyurethanes, ethylene-vinyl acetate copolymers, polyethylene, and polypropylene, thermoplastic elastomers such as hydrogenated styrene-butadiene-styrene copolymers, styrene-isoprene-styrene copolymers or hydrogenated products thereof, and mixtures of a thermoplastic elastomer and a softener such as polyolefins, and ethylene-ethyl acrylate. The flexible resin is preferably soft polyvinyl chloride, polyurethanes, ethylene-vinyl acetate copolymers, polyolefins, and thermoplastic elastomers including any of these as the main component, more preferably soft polyvinyl chloride and polyolefins, and further preferably soft polyvinyl chloride. Soft polyvinyl chloride is preferable for the container for the blood processing filter, because improvements of plasticizers used for the synthesis have advanced in recent years to result in the distribution of the materials using plasticizers with higher safety in the market.

When the filtration medium has a shape of, for example, flat plate, the shape of the container can be a flat shape of a polygon, such as quadrangle or hexagon, or circle or oval according to the shape thereof (for example, Figure 1 and Figure 2). When the filtration medium is cylindrical, the container is also preferably cylindrical.

[Filtration medium]

The filtration medium preferably includes a fiber. The fiber is preferably in the form of a nonwoven fabric. The nonwoven fabric preferably has one or more filter layers. When the filtration medium includes a plurality of filter layers, a plurality of the filter layers can be the same or different from each other.

The fiber included in the filtration medium is preferably a polyester fiber. The polyester preferably contains an oligomer having the terephthalic acid skeleton, but not particularly limited thereto. Specifically, polyester resins such as polybutylene terephthalate (PBT) and polyethylene terephthalate (PET) are preferable. The polyester fiber can be formed from only a single kind of material, or can be formed from a plurality of kinds of materials.

For example, as shown in Figures 1 and 2, when the filter is made by gripping a filtration medium including a plurality of kinds of fibers with a grasp using two parts, i.e., the container component on the outlet side and the container component on the inlet side composing a rigid container, use of the fibers with a high degree of crystallinity disposed at the closest position to the container component on the outlet side enables firmer grasp of the filtration medium with the grip part of the container component on the outlet side after steam heat treatment. By this, the phenomenon in which blood passes between the grip parts and the filtration medium without penetrating the filtration medium and directly flows from the inlet space to the outlet space (side leak phenomenon), can be prevented, thereby enhancing the capability to remove leukocyte and the like.

[Coating layer]

The fiber included in the filtration medium can have a coating layer on the surface thereof. Herein, the fiber that does not have a coating layer is also referred to as the "fiber base material".

The coating layer preferably includes, for example, a copolymer having a monomer unit having a nonionic hydrophilic group and a monomer unit having a basic nitrogen-containing functional group. When the copolymer having a basic nitrogen-containing functional group is used, the fiber surface can be charged with positive electrons by the coating treatment, and the affinity to leukocytes can also be enhanced.

Examples of the monomer unit having a nonionic hydrophilic group include monomer units derived from 2-hydroxyethyl(meth)acrylate, 2-hydroxypropyl(meth)acrylate, vinyl alcohols, (meth)acrylamide, and N-vinylpyrrolidone. In view of availability, ease of handling when polymerizing, and performance when blood is allowed to flow, the monomer unit having a nonionic hydrophilic group is preferably a monomer unit derived from 2-hydroxyethyl(meth)acrylate. The monomer unit derived from a vinyl alcohol is typically generated by hydrolysis after polymerization of vinyl acetate.

Examples of the monomer unit having a basic nitrogen-containing functional group include monomer units derived from derivatives of (meth)acrylic acid such as diethylaminoethyl(meth)acrylate, dimethylaminoethyl(meth)acrylate, dimethylaminopropyl(meth)acrylate, 3-dimethylamino-2-hydroxypropyl(meth)acrylate; styrene derivatives such as p-dimethylaminomethylstyrene, and p-diethylaminoethylstyrene; vinyl derivatives of nitrogen-containing aromatic compounds such as 2-vinylpiridine, 4-vinylpiridine, and 4-vinylimidazole; and derivatives in the form of a quaternary ammonium salt obtained from the above vinyl compounds using alkyl halide or the like. In view of availability, ease of handling when polymerizing, and performance when blood is allowed to flow, the monomer unit having a basic nitrogen-containing functional group is preferably monomer unit derived from diethylaminoethyl(meth)acrylate and dimethylaminoethyl(meth)acrylate.

The method for forming a coating layer on the fiber base material is not particularly limited, and examples thereof include a method including immersing the fiber base material in a coating solution containing a monomer and/or

a polymer (copolymer) and, as needed, a solvent and others and then appropriately removing the coating solution (dipping method), and a method including contacting the fiber base material with a roll into which a coating solution is immersed (transfer method).

The fiber preferably has a basic nitrogen-containing functional group on the circumferential surface thereof. The circumferential surface of the fiber means the entire part of the fiber exposed to the outside. Accordingly, when the fiber has a coating layer, the circumferential surface is the surface of the coating layer. When the fiber does not have a coating layer, the circumferential surface means the surface of the fiber base material. When a basic nitrogen-containing functional group is present on the circumferential surface of the fiber, the basic nitrogen-containing functional group has positive electrons in blood, thereby increasing the affinity between the fiber and leukocytes in blood to enhance the capability to remove leukocyte.

The fiber can have a nonionic hydrophilic group on the circumferential surface thereof. When a nonionic hydrophilic group is present on the circumferential surface of the fiber, the wettability of the fiber surface to blood increases to increase the effective filtration area (area actually used for filtration) of the fiber, and consequently both of the reduction in filtration time and the enhanced capability to remove leukocyte and the like can be advantageously obtained.

Examples of the method for allowing the nonionic hydrophilic group and the basic nitrogen-containing functional group to be present on the circumferential surface of the fiber include, for the fiber having a coating layer, a method including coating the fiber with a coating material containing a monomer and/or a polymer including the nonionic hydrophilic group and the basic nitrogen-containing functional group, and for the fiber having no a coating layer, a method including spinning a fiber material containing a monomer and/or a polymer including the nonionic hydrophilic group and the basic nitrogen-containing functional group.

The ratio of the amount of substance of the basic nitrogen-containing functional group to the total amount of substances of the nonionic hydrophilic group and the basic nitrogen-containing functional group in the circumferential surface of the fiber is preferably 0.2 to 50.0 mole percent, more preferably 0.25 to 10 mole percent, further preferably 1 to 5 mole percent, and most preferably 2 to 4 mole percent. The contents of the basic nitrogen-containing functional group and the nonionic hydrophilic group can be measured by the analysis by NMR, IR, TOF-SIMS and the like. The thus controlled ratio of the basic nitrogen-containing functional group and the nonionic hydrophilic group enables the efficient removal of leukocytes and others with the assurance of stable wettability to blood while preventing unnecessary clogging with blood components such as platelet.

When the fiber includes a coating layer on the circumferential surface thereof, the components including the basic nitrogen-containing functional group and the nonionic hydrophilic group and forming the coating layer can be causative substances for increasing the absorbance defined by ISO 1135-4. Accordingly, for keeping the absorbance at a certain level or less, the mass of the coating layer per g of the total of the fiber base material and the coating layer is preferably 15 mg or less, more preferably 10 mg or less, and further preferably 5 mg or less.

The mass of the coating layer can be calculated by, for example, the following procedure. The fiber base material before supporting the coating material is dried for 1 hour in a dryer at 60°C, and then allowed to stand for 1 hour in a desiccator, and the mass (A g) is measured. In the same manner, the fiber base material supporting the coating material is dried for 1 hour in a dryer at 60°C, and then allowed to stand for 1 hour in a desiccator, and the mass (B g) is measured. The mass of the coating layer is calculated by the following equation.

$$\text{Mass of the coating layer per gram of the total of the fiber base material and the coating layer (mg/g)} = (B-A) \times 1000/B$$

[Specific surface area]

The specific surface area of the fiber base material or coating layer-containing fiber included in the filtration medium is preferably 0.6 to 2.5 $m^2/g$, more preferably 0.8 to 2.0 $m^2/g$, and further preferably 1.0 to 1.5 $m^2/g$. The method for measuring a specific surface area is as described in Examples.

[Surface area of filtration medium]

The surface area of the filtration medium (total surface area of the fiber base material or the coating layer-containing fiber) is preferably 6.0 $m^2$ or more, more preferably 7.0 $m^2$ or more, and further preferably 8.0 $m^2$ or more, in view of enhancing the leukocyte removing performance. When the blood processing filter includes a plurality of the filtration media, the surface area is the total of a plurality of the filtration media. When the surface area of the filtration

medium is too large, the leukocyte removing performance reaches a plateau, and only the maximum absorbance (amount of monomer components and oligomer components) of an aqueous extract of the blood processing filter in the range from 240 to 245 nm increases, and it is thus preferable that the surface area of the filtration medium be, for example, 10.0 $m^2$ or less, 9.0 $m^2$ or less, and 8.5 $m^2$ or less. The method for measuring the surface area of the filtration medium is as described in Examples.

[Maximum absorbance]

The maximum absorbance of an aqueous extract of the blood processing filter in the range from 240 to 245 nm is preferably 0.03 or less, more preferably 0.02 or less, and further preferably 0.01 or less. The maximum absorbance of 0.03 or less means a sufficiently small amount of monomers component and oligomer components included in the blood processing filter. The method for measuring the maximum absorbance is as described in Examples. In Examples, the maximum absorbance is measured by using three blood processing filters, but the above numerical values are of one blood processing filter.

In view of reducing the amount of elutable substances while maintaining the leukocyte removing performance, the maximum absorbance per unit mass (g) of the filtration medium (unit mass (g) of the fiber base material or coating layer-containing fiber) is preferably 0.003 $g^{-1}$ or less, more preferably 0.002$g^{-1}$ or less, and further preferably 0.001$g^{-1}$ or less.

[Residue on evaporation]

The amount of the residue on evaporation from an aqueous extract of the blood processing filter is preferably 1.7 mg or less, more preferably 0.8 mg or less, further preferably 0.5 mg or less, in view of reducing the amount of elutable substances from the filter. The method for measuring a residue on evaporation is as described in Examples. In Examples, the residue on evaporation is measured by using three blood processing filters, but the above numerical values are of one blood processing filter.

[Average fiber diameter]

The average fiber diameter of the filtration medium is preferably 0.1 to 1.6 μm, more preferably 0.5 to 1.5 μm, and further preferably 0.9 to 1.4 μm. When the average fiber diameter is 1.6 μm or less, the leukocyte removing performance can be more enhanced.

The average fiber diameter refers to the value determined in accordance with the following method. Specifically, parts recognized as substantially uniform were sampled from a single sheet or a plurality of fibrous material sheets composing the filtration medium and photographed using a scanning electron microscope or the like. For sampling, an effective filtration cross-sectional area of the fibrous materials is divided into squares 0.5 cm on a side, of which 6 squares were randomly sampled. For random sampling, for example, the above squares are numbered, and then divisions of required squares can be selected by a method involving use of a random number table or the like. Additionally, three or more places, and preferably five or more places, of the sampled division is photographed at a magnification of 2500x. Each of the sampled divisions is photographed at the center part and near parts thereof until the total number of fibers photographed exceeds 100. The value obtained by dividing the sum of diameters of all fibers measured by the number of fibers is defined as the average fiber diameter. The diameter herein refers to the width of a fiber in a perpendicular direction to the fiber axis. However, a plurality of fibers may overlap so that the width of a fiber cannot be measured because a fiber is behind other fibers, or a plurality of fibers may melt to thereby form a thick fiber, or further fibers having notably different diameters may be mixed. Data in such cases are deleted.

[Pressure loss in ventilation]

The pressure loss of the blood processing filter in ventilation is preferably 100 to 2000 Pa, more preferably 200 to 1800 Pa or 300 to 2000 Pa, and further preferably 400 to 1600 Pa. The pressure loss in ventilation is a measure of the resistance of the filter to an air flow, and the filtration performances such as the capability to remove leukocyte and the like can be evaluated by the pressure loss in ventilation. Specifically, when the pressure loss in ventilation is large, the capability to remove leukocyte is large. The pressure loss in ventilation can be increased by, for example, giving a smaller fiber diameter of the filtration medium.

The pressure loss in ventilation can be determined by measuring a pressure loss (Pa) of the air caused in the filter when dry air at a constant air flow (3.0 ml/min) is allowed to pass through the filter. The pressure loss can be measured with, for example, a Cosmo DP gauge (model type: DP-320B).

[Capability to remove leukocyte]

When 400 mL of a leukocyte-containing liquid having a leukocyte concentration of 5000 cells/μL is filtered, the leukocyte removing performance of the blood processing filter represented by a formula below is preferably 5.3 or more, more preferably 4.3 or more, and further preferably 3.3 or more.

Leukocyte removing performance = −log [(leukocyte concentration in leukocyte-containing liquid after filtration)/(leukocyte concentration in leukocyte-containing liquid before filtration)]

When 400 mL of a leukocyte-containing liquid having a leukocyte concentration of 5000 cells/μL is filtered through the blood processing filter, the residual white blood cell count is preferably less than $10^6$ cells (6 Log), more preferably less than $10^{5.8}$ cells (5.8 Log), further preferably less than $10^{5.5}$ cells (5.5 Log), and particularly preferably less than $10^{5.3}$ cells (5.3 Log).

<Method for producing blood processing filter>

One of the present embodiments relates to a method for producing the blood processing filter. The fiber base material included in the filtration medium can be produced by either a wet method or a dry method. A melt blown method is preferably used, but not particularly limited thereto.

In the melt blown method, a molten polymer stream melted inside an extruder is filtered through an appropriate filter, then guided to a part for introducing the molten polymer of a melt blown die, and subsequently discharged from an orifice nozzle. A hot gas introduced to a part for introducing hot air at the same time is guided to a slit for ejecting hot air formed by the melt blown die and a lip and ejected therefrom, thereby to make the molten polymer discharged as above thinner to form ultra-fine fibers, and the formed ultra-fine fibers are laminated to obtain the fiber. Further, heat treatment of the fiber using a heat suction drum, a hot plate, hot water, a hot air heater, and the like brings about the effect of increasing the thermal stability of the fiber. Accordingly, when the leukocyte removing filter including the fiber is sterilized with a high-temperature high-pressure steam before use, the fiber shrinkage inside the filter can be prevented and distortions and deformations of the filter itself can thus be prevented.

In the method for producing the blood processing filter according to the present embodiment, the step for achieving the maximum absorbance of an aqueous extract of the blood processing filter in the range from 240 to 245 nm of 0.03 or less (the step for decreasing the amount of monomer components and oligomer components) may be, for example, any of the following steps. These steps can be used singly, or used in combination.

(1) Washing step
(2) Spinning step under a low temperature condition
(3) Vacuum venting step

[Washing step]

The washing step is a step of pre-removing elutable substances (monomer components and oligomer components) from the blood processing filter by washing the fiber included in the filtration medium. Examples of the washing solution include water, ethanol, and a mixed solvent thereof, and in view of hydrophilizing the fiber surface, a mixed solvent of ethanol and water (hereinafter referred to as the "ethanol aqueous solution") is preferable. The washing method is not particularly limited and, examples thereof include the immersion of the fiber in the washing solution. Ethanol is a combustible substance and thus is preferably used at an ethanol concentration as low as possible in view of preventing an ignition explosion.

The concentration of the ethanol aqueous solution is preferably 12% (v/v) or more, more preferably 20% (v/v) or more, further preferably 30% (v/v) or more, and most preferably 40% (v/v) or more, in view of washing efficiency. Considering the safety, the upper limit of the concentration of the ethanol aqueous solution can be, for example, 75% (v/v), and 65% (v/v).

The temperature of the ethanol aqueous solution is preferably 20°C or more, more preferably 30°C or more, and further preferably 40°C or more, in view of washing efficiency. The upper limit of the temperature of the ethanol

aqueous solution can be, for example, 70°C and 60°C.

The present inventors have found that the maximum absorbance in the range from 240 to 245 nm is reduced by about 90% when a PBT fiber is immersed for 60 minutes in a 45% (v/v) ethanol aqueous solution heated to 40°C. The present inventors also have found that as the concentration and the temperature of the ethanol aqueous solution are higher, the absorbance reduction is commonly more significant, and also that the temperature of the ethanol aqueous solution contributes to the absorbance reduction more than the concentration thereof. These suggest that the monomer components and the oligomer components included in the fiber have the solubility that increases in a temperature-dependent manner.

[Spinning step under low-temperature condition]

The spinning step under a low-temperature condition is a step of spinning under a condition that the melting temperature of the fiber source (resin) is lower than the typical temperature. The melting temperature refers to a temperature at the time of extruding the resin from a spinneret when melt spinning. The melting temperature for a PBT resin is typically about 260 to 310°C, but when this temperature is reduced, the pyrolysis of a resin can be prevented to thereby decrease the amount of substances (monomer components and oligomer components) to be eluted from the blood processing filter. When the melting temperature is simply reduced, the melt viscosity of the resin may be reduced to thereby increase the average fiber diameter of the fiber and therefore reduce the surface area. In such an instance, a smaller diameter can be achieved by increasing a hot air pressure, which is another parameter.

Specifically, the spinning step under a low-temperature condition can be carried out in the following manner.

A typical die temperature when melt spinning the fiber is reduced by 20 to 30°C. Here, when the resin is solidified, shots (resin grains) are likely to be mixed in the fiber and hence the temperature reduction is halted before shots are produced.

A hot air pressure is increased as needed to achieve a smaller average fiber diameter of the fiber. A hot air pressure is increased as measuring a fiber diameter until the average fiber diameter reaches an equivalent average fiber diameter of the fiber typically spun.

The present inventors have made nonwoven fabrics having the same average fiber diameters of a PBT resin having a melting point of 220°C under two conditions: a condition of a melting temperature of 280°C and a hot air pressure of 0.30 MPa; and a condition of a melting temperature of 260°C and a hot air pressure of 0.40 MPa. In these instances, it is found that the maximum absorbance reduces by about 5% in the range from 240 to 245 nm when the melting temperature is 260°C, compared with the case when the melting temperature is 280°C. This comparison has been carried out under a condition of 260°C and a condition of 280°C on the premise that the specific surface areas of the fiber itself were equivalent.

The melting temperature is suitably adjusted in accordance with the kind of a resin to be used, and is preferably the melting point of the resin +20°C to the melting point of the resin +90°C, more preferably the melting point of the resin +30°C to the melting point of the resin +80°C, and further preferably the melting point of the resin +40°C to the melting point of the resin +70°C.

The hot air pressure is suitably adjusted in accordance with the kind of a resin to be used, and is preferably 0.20 to 0.50 MPa, more preferably 0.22 to 0.45 MPa, and further preferably 0.25 to 0.40 MPa.

[Vacuum venting step]

Vacuum venting step is a step of suctioning and removing a volatile component released from the resin melting under the vacuum condition while melting the resin after crystallization treatment and immediately before use for spinning by passing the resin through an extruder. The preset temperature needs to be optimized depending on the resin, but the temperature is preferably preset as high as possible. In this step, substances (monomer components and oligomer components) elutable from the blood processing filter vaporize and thus the concentration thereof in the resin reduces. As a result the monomer components and the oligomer components in the fiber reduce, thereby reducing the maximum absorbance in the range from 240 to 245 nm.

Specifically, the vacuum venting step can be carried out in the following manner.

The dried resin is fed to an extruder using a gear pump. The melting temperature during this operation is the melting point of the resin +10°C or more.

In the extruder, the temperature is preset to a temperature more than a melting point of the resin, heat treatment is carried out on the resin in a melted state, and the air in the extruder is evacuated while retaining the resin. The retention time is primarily determined by the structure of an extruder and the discharge of the nonwoven fabric but is preferably preset from 30 seconds to 5 minutes.

For the air evacuation, it is desirable to increase a degree of vacuum to effectively carry out the suction and removal of the volatile component, and the evacuation is carried out at a degree of vacuum of at least -0.01 MPa or

more. It is preferably -0.03 to -0.10 MPa, more preferably -0.05 to -0.10 MPa, and further preferably - 0.07 to -0.10 MPa.

When a melting temperature is too low, a vapor pressure of the volatile component is low and the removal effect is accordingly low. However, when a melting temperature is too high, the resin is burnt to generate black spots on the nonwoven fabric, which is not preferable. For this reason, the melting temperature is preferably the melting point of the resin +20°C to the melting point of the resin +90°C, more preferably the melting point of the resin +30°C to the melting point of the resin +80°C, and further preferably the melting point of the resin +40°C to the melting point of the resin +70°C.

When the present inventors have made a nonwoven fabric of a PBT resin having a melting point of 220°C by carrying out the vacuum venting at a treatment temperature of 255°C and a degree of vacuum of -0.05 MPa for a retention time of 2 minutes, it has been confirmed that the maximum absorbance in the range from 240 to 245 nm reduced by about 30% compared with the case of no vacuum degree (air suction in the extruder is not carried out.)

<Method for removing leukocyte>

One of the present embodiments relates to a method for removing leukocyte, the method comprising the step of passing the leukocyte-containing liquid through the blood processing filter to thereby remove leukocytes from a leukocyte-containing liquid. The passing achieves a leukocyte residual rate of preferably $10^{-3}$ or less, more preferably $10^{-4}$ or less, further preferably $10^{-5}$ or less, and particularly preferably $10^{-6}$ or less. Additionally or alternatively, the passing achieves a residual white blood cell count in 400 mL of a leukocyte-containing liquid of preferably less than $10^{6}$ cells (6 Log), more preferably less than $10^{5.8}$ cells (5.8 Log), further preferably less than $10^{5.5}$ cells (5.5 Log), and particularly preferably less than $10^{5.3}$ cells (5.3 Log).

Herein, the leukocyte-containing liquid collectively refers to body fluids including leukocytes and synthetic bloods and specifically refers to: whole blood and liquids including a single kind or a plurality of kinds of blood components obtained and prepared from whole blood, including whole blood, packed red blood cell solution, washed red blood cell suspension, frozen-thawed red blood cell concentrate, synthetic blood, platelet poor plasma (PPP), platelet rich plasma (PRP), blood plasma, frozen blood plasma, platelet concentrate, and buffy coat (BC); or solutions obtained by adding an anticoagulant agent or a preservation solution to any of these liquids; or whole blood preparations, erythrocyte preparations, thrombocyte preparations, blood plasma preparations or the like.

Liquids obtained by treating the above liquids by the method of the present embodiment are referred to as the liquids from which leukocytes are removed.

Hereinafter, an embodiment of the method for preparing a blood preparation by removing leukocytes by the method for removing leukocyte will be described.

(Preparation of leukocyte-removed whole blood preparation)

A whole blood preparation is provided by adding, to collected whole blood, a preservation solution such as Citrate Phosphate Dextrose (CPD), Citrate Phosphate Dextrose Adenine-1 (CPDA-1), Citrate Phosphate-2-Dextrose (CP2D), Acid Citrate Dextrose Formula-A (ACD-A), Acid Citrate Dextrose Formula-B (ACD-B), and heparin, and an anticoagulant agent, for example, and then leukocytes are removed from this whole blood preparation using the blood processing filter of the present embodiment, whereby the leukocyte-removed whole blood preparation can be obtained.

In the preparation of the leukocyte-removed whole blood preparation, when removing leukocytes before stored, the leukocyte-removed whole blood preparation can be obtained by removing leukocytes at room temperature or under refrigeration using the blood processing filter from whole blood stored preferably at room temperature or under refrigeration within 72 hours, further preferably within 24 hours, particularly preferably within 12 hours, and most preferably within 8 hours, from collection. In the case of removing leukocytes after stored, leukocytes may be removed from whole blood stored at room temperature or under refrigeration or freezing preferably within 24 hours before use using the blood processing filter to obtain the leukocyte-removed whole blood preparation.

(Preparation of leukocyte-removed erythrocyte preparation)

To collected whole blood, a preservation solution such as CPD, CPDA-1, CP2D, ACD-A, ACD-B, and heparin and an anticoagulant agent are added. The separation method of each blood component includes a method including removing leukocytes from whole blood and subsequently carrying out centrifugation, and a method including centrifuging whole blood and subsequently removing leukocytes from red blood cells or red blood cells and BC.

In the case of removing leukocytes from whole blood and subsequently carrying out centrifugation, the leukocyte-removed erythrocyte preparation can be obtained by centrifuging the leukocyte-removed whole blood.

For the case of centrifuging whole blood before removing leukocytes, there are two types of centrifugation conditions: a weak centrifugation condition which separates red blood cells from PRP; and a strong centrifugation

condition which separates red blood cells and BC from PPP. A preservation solution such as SAGM, AS-1, AS-3, AS-5, and MAP may be added as needed to red blood cells, or red blood cells including BC, which are separated from whole blood, and then leukocytes may be removed from red blood cells using the leukocyte removing filter to obtain the leukocyte-removed erythrocyte preparation.

In the preparation of the leukocyte-removed erythrocyte preparation, whole blood stored preferably at room temperature or under refrigeration can be centrifuged within 72 hours, further preferably within 48 hours, particularly preferably within 24 hours, and most preferably within 12 hours, from collection.

In the case of removing leukocytes before stored, leukocytes may be removed from an erythrocyte preparation stored preferably at room temperature or under refrigeration within 120 hours, further preferably within 72 hours, particularly preferably within 24 hours, and most preferably within 12 hours, from collection using the blood processing filter at room temperature or under refrigeration to obtain the leukocyte-removed erythrocyte preparation. In the case of removing leukocytes after stored, leukocytes may be removed using the blood processing filter within 24 hours before use from an erythrocyte preparation stored preferably at room temperature, under refrigeration or freezing to obtain the leukocyte-removed erythrocyte preparation.

(Preparation of leukocyte-removed thrombocyte preparation)

To collected whole blood, a preservation solution such as CPD, CPDA-1, CP2D, ACD-A, ACD-B, and heparin and an anticoagulant agent are added.

The separation method of each blood component includes a method including removing leukocytes from whole blood and subsequently carrying out centrifugation, and a method including centrifuging whole blood and subsequently removing leukocytes from PRP or platelets.

In the case of removing leukocytes from whole blood and subsequently carrying out centrifugation, the leukocyte-removed thrombocyte preparation can be obtained by centrifuging the leukocyte-removed whole blood.

In the case of centrifuging whole blood before removing leukocytes, there are two types of centrifugation conditions; a weak centrifugation condition which separates red blood cells from PRP; and a strong centrifugation condition which separates red blood cells and BC from PPP. In the case of the weak centrifugation condition, leukocytes may be removed from PRP separated from whole blood using the blood processing filter and subsequently the leukocyte-removed thrombocyte preparation may be obtained by centrifugation, or PRP may be centrifuged to separate platelets from PPP and subsequently leukocytes may be removed by the blood processing filter to obtain the leukocyte-removed thrombocyte preparation. In the case of the strong centrifugation condition, a preservation solution, blood plasma and others may be added as needed to a pool of one unit or several to dozen units of BC separated from whole blood, centrifugation may be carried out thereby to obtain platelets, and leukocytes may be removed from the obtained platelets by the blood processing filter to obtain the leukocyte-removed thrombocyte preparation.

In the preparation of the leukocyte-removed thrombocyte preparation, whole blood stored preferably at room temperature is centrifuged within 24 hours, further preferably within 12 hours, and particularly preferably within 8 hours, from collection. In the case of removing leukocytes before stored, leukocytes may be removed at room temperature using the blood processing filter from the thrombocyte preparation stored preferably at room temperature within 120 hours, further preferably within 72 hours, particularly preferably within 24 hours, and most preferably within 12 hours, from collection to obtain the leukocyte-removed thrombocyte preparation. In the case of removing leukocytes after stored, leukocytes may be removed from the thrombocyte preparation stored preferably at room temperature or under refrigeration or freezing within 24 hours before use using the blood processing filter to obtain the leukocyte-removed thrombocyte preparation.

(Preparation of leukocyte-removed blood plasma preparation)

To the collected whole blood, a preservation solution such as CPD, CPDA-1, CP2D, ACD-A, ACD-B, and heparin and an anticoagulant agent are added.

The separation method of each blood component includes a method including removing leukocytes from whole blood and subsequently carrying out centrifugation and a method including centrifuging whole blood and subsequently removing leukocyte from PPP or PRP.

In the case of carrying out centrifugation after removing leukocytes from whole blood, the leukocyte-removed whole blood may be centrifuged to obtain the leukocyte-removed blood plasma preparation.

In the case of centrifuging whole blood before removing leukocytes, there are two types of centrifugation conditions: a weak centrifugation condition which separates red blood cells from PRP; and a strong centrifugation condition which separates red blood cells and BC from PPP. In the case of the weak centrifugation condition, leukocytes may be removed from PRP using the blood processing filter and subsequently the leukocyte-removed blood plasma preparation may be obtained by centrifugation. Alternatively, PRP may be centrifuged to separate platelets from PPP

and subsequently leukocytes may be removed by the blood processing filter to obtain the leukocyte-removed blood plasma preparation. In the case of the strong centrifugation condition, leukocytes may be removed from PPP by the blood processing filter to obtain the leukocyte-removed blood plasma preparation.

In the preparation of the leukocyte-removed blood plasma preparation, whole blood stored preferably at room temperature or under refrigeration can be centrifuged within 72 hours, further preferably within 48 hours, particularly preferably within 24 hours, and most preferably within 12 hours, from collection. Leukocytes may be removed from a blood plasma preparation stored preferably at room temperature or under refrigeration within 120 hours, further preferably within 72 hours, particularly preferably within 24 hours, and most preferably within 12 hours, from collection using the blood processing filter at room temperature or under refrigeration to obtain the leukocyte-removed blood plasma preparation. In the case of removing leukocytes after stored, leukocytes may be removed using the blood processing filter within 24 hours before use from a blood plasma preparation stored preferably at room temperature, under refrigeration or freezing to obtain the leukocyte-removed blood plasma preparation.

The form from blood collection to the preparation of the leukocyte-removed blood preparations can be any form, and examples thereof include: collecting blood using a blood-collecting needle connected to a whole-blood container, and connecting the container containing whole blood or centrifuged blood components and the blood processing filter to carry out the leukocyte removal; or collecting blood in a circuit in which at least a blood-collecting needle, a blood container, and the blood processing filter are aseptically connected, and carrying out the leukocyte removal before or after centrifugation; or carrying out the leukocyte removal by connecting the blood processing filter to a container containing blood components obtained by an automatic blood collector or by the pre-connected blood processing filter. However, the present embodiment is not limited to these forms. Further, whole blood may be centrifuged to each component using an automatic blood component collector, a preservation solution may be added as needed, and then either red blood cells, red blood cells including BC, BC, platelets, PRP, or PPP may be immediately passed through the blood processing filter to remove leukocytes, thereby obtaining the leukocyte-removed erythrocyte preparation, leukocyte-removed thrombocyte preparation, or leukocyte-removed blood plasma preparation.

In the present embodiment, the leukocyte removal can be carried out by allowing a leukocyte-containing blood to flow through the blood processing filter via a tube using a difference in elevation from a container containing the leukocyte-containing liquid placed at a position higher than the blood processing filter, or can be carried out by allowing leukocyte-containing blood to flow by applying a pressure by means of a pump or the like from the inlet side of the blood processing filter and/or reducing a pressure from the outlet side of the blood processing filter.

## Example

Hereinafter, the present invention will be described by way of Examples but is not limited to the following Examples. Performances of the blood processing filters were measured by the following methods.

### <Specific surface area>

The specific surface area herein refers to the surface area per gram of the fiber base material or the coating layer-containing fiber, and is the value measured by the BET adsorption method using krypton as an adsorption gas, and it can be measured using a TriStar II 3020 analyzer manufactured by SHIMADZU CORPORATION.

Specifically, the specific surface area is measured by the following procedure.

1) A certain length of the fiber with a width of 3.5 cm was cut out, and two sheets are stacked. The length is adjusted so that the mass of the two fiber sheets is 0.64 to 0.70 g.
2) The fiber sheets are wound and put in a cell, and dried at 60°C for 40 minutes, and then a nitrogen gas is allowed to flow for 30 seconds to carry out gas replacement. Subsequently, the mass of the entire cell is measured, and the mass of the cell's tare is subtracted to measure the mass of the treated fiber.
3) After a blank-run using a blank cell, the cell containing the fiber is mounted in the analyzer, a krypton gas is allowed to flow to carry out the measurement, and the specific surface area is calculated.

As the specific surface area of the fiber is larger, the area capable of adsorbing cells, plasma proteins and the like is larger when blood is processed using a filtration medium including the fiber having the same mass.

### <Surface area of filtration medium>

The surface area of the filtration medium is calculated by the specific surface area of the fiber base material or coating layer-containing fiber ($m^2/g$) × the mass of the fiber used for the effective filtration part.

Specifically, for example, 10 sheets of a fiber having a specific surface area of 1.5 $m^2/g$ and a mass per unit

area of 40 g/m$^2$ may be stuck and cut so that an effective filtration area of each fiber is 45 cm$^2$ to thereby make a filtration medium, and in this case, the surface area of the filtration medium is as follows.

Specific surface area of a fiber of 1.5 (m$^2$/g) × mass per unit area of 40 (g/m$^2$) × 10 sheets × effective filtration area of 45 cm2/10000 = 2.7 m$^2$

<Pressure loss in ventilation>

The pressure loss in ventilation was measured as a pressure loss using a DP gauge when an air was allowed to pass through the blood processing filter in a flow rate of about 3.0 litter/min for 20 to 30 seconds at room temperature of 20 to 25°C.

<Absorbance>

[Elution test in accordance with ISO 1135-4 (2015)]

For the purified water to be used for the evaluation, 250 mL of Elix water, which was prepared using an ultrapure water purification system Milli-Q (Millipore) and equivalent to ISO 3696 Grade 2, was used.

ISO 1135-4 is the standard required for blood transfusion set standard, and accordingly three blood processing filters that was subjected to steam heat treatment as the sterilization treatment and included a flexible container were provided. As shown in Figure 3, the inlets and the outlets of the filters were connected in series using vinyl chloride tubes of 10 cm having an inner diameter of 2.9 mm and an outer diameter of 4.2 mm, and further the inlet of the first filter was connected using a 50 cm-tube, which was allowed to pass through an electric pump so that Elix water circulated. Then the tip of the tube was put at the bottom of an Erlenmeyer flask in which 250 mL of Elix water was contained. The outlet of the third filter was similarly connected using a 50 cm-tube, which was fixed at the spout of the Erlenmeyer flask. Finally, the Erlenmeyer flask was immersed in a thermostatic bath at 37°C, and the temperature was maintained.

The flow volume of the electric pump was preset to 1 L/hr to start the circulation, the inlet-side tube was taken out from the Erlenmeyer flask 2 hours later, and the liquid in the circuit was recovered in an Erlenmeyer flask using the pump and filtered using a filter paper thereby to obtain an aqueous extract. The recovered aqueous extract was lightly shaken and used as the test liquid. Further, for a blank test solution, a liquid similarly circulated for 2 hours in a circuit in which the filters were not connected but only tubes were connected was recovered, filtered using a filter paper, and used.

Chinese Industry Standard (YY) has the same extraction method as ISO 1135-4, except that the circulation extract is carried out using 350 mL unlike the ISO Standards.

[UV Measurement]

UV Measurement was also carried out under the conditions described in ISO 1135-4 B.6. However, the measurement range was 190-320 nm for measuring in a low wavelength range. Other measurement conditions are as follows. A measuring instrument used was a UV visible near-infrared spectroscopy (manufactured by JASCO, V-770 Spectrophotometer). A base line was first made only using the blank test solution and then subtracted from the subsequent UV absorption value of the test solution thereby to calculate an UV absorption value of the test solution.

(Measurement conditions)

- Measurement range: 190-320 nm
- Data import interval: 1 nm
- UV/Vis Band width: 5.0 nm
- NIR Band width: 20.0 nm
- UV/Vis Response: 0.06 sec
- NIR Response: 0.06 sec
- Scanning speed: 20 nm/min

(Pass-Fail Criteria)

According to the definition of ISO, the maximum absorbance in the range from 250 to 320 nm must be 0.1 or less (ISO 1135-4 6.5 and B.6), and it is preferable to be 0.05 or less when considering variations of each filter. In the above measurement, three blood processing filters were used and hence the maximum absorbance per filter is 1/3 of

the above numerical value.

In relation to the monomer component and the oligomer component of interest in the present invention, the maximum absorbance in the range from 240 to 245 nm is measured and the maximum absorbance per filter is preferably 0.03 or less.

<Residue on evaporation>

The measurement of the residue on evaporation was carried out under the following conditions. The conditions clearly described in ISO 1135-4 6.4 were referenced as they are.

First, a glass weighing dish having a volume of 74 mL or more was dried at 105°C for 1 hour in a dryer, and cooled at normal temperature in a desiccator for 1 hour, and then the mass was quickly measured using an electronic weighing scale (manufactured by Mettler-Toledo). Subsequently, 50 mL of the aqueous extract after filtration obtained in the above <Absorbance> section was weighed in a graduated cylinder, put in the weighing dish after measuring the mass, and heated until evaporated to dryness in a boiling water bath. The glass weighing dish after evaporation was dried at 105°C for 1 hour in a dryer, and cooled at normal temperature in a desiccator for 1 hour, and then the mass was quickly measured using an electronic weighing scale (manufactured by Mettler-Toledo). A mass difference between before and after treatment of the glass weighing dish was measured. Similarly, a mass difference between before and after treatment using the blank test solution was measured. A value obtained by subtracting the mass difference of the blank test solution from the mass difference of the test solution was defined as the residual amount on evaporation of the test solution.

(Pass-Fail Criteria)

According to the definition of ISO, the residue on evaporation must be 5 mg or less (ISO 1135-4 6.4), and it is preferably 2.5 mg or less when considering variations of each filter. When the Chinese Industry Standard (YY) having the pass-fail criteria of 2.0 mg or less is considered, the residue on evaporation is further preferably 1.5 mg or less. In the above measurement, three blood processing filters were used, and hence the residue on evaporation per filter is 1/3 of the above numerical value.

<Evaluation of Capability to remove leukocyte / evaluation of filtration time>

For the blood to be used for the evaluation, whole blood was used that was obtained by adding 56 mL of a CPD solution, which is an anticoagulant agent, to 400 mL of blood immediately after collected, mixing them, and allowing the resultant to stand for 7 hours. Hereinafter, this blood prepared for the blood evaluation is referred to as the blood before filtration.

However, in the blood transfusion market, blood can be used as room temperature-stored blood or refrigeration stored blood, and hence the evaluation this time was carried out under the room temperature-stored blood condition, which causes an increased residual white blood cell count, as the worst case.

A blood bag filled with blood before filtration and the inlet of the blood processing filter after steam heat treatment were connected using a 40 cm-vinyl chloride tube having an inner diameter of 3 mm and an outer diameter of 4.2 mm. Further, the outlet of the blood processing filter and a blood bag for recovery were similarly connected using an 85 cm-vinyl chloride tube having an inner diameter of 3 mm and an outer diameter of 4.2 mm. Subsequently, the blood before filtration was allowed to flow through the blood processing filter at a difference in elevation of 140 cm from the upper part of the blood bag filled with the blood before filtration, and the filtration time required for the amount of blood flowing in the blood bag for recovery to reach 1.0 g/min was measured.

Further, 3 mL of blood was recovered from the blood bag for recovery (hereinafter, referred to as the blood after filtration). The capability to remove leukocyte was evaluated by determining the residual white blood cell count. The white blood cell count of the blood after filtration was measured using the flow cytometry method (device: manufactured by BECTON DICKINSON, FACSCanto) to calculate the residual white blood cell count using the formula below. The measurement of white blood cell count was carried out by sampling 100 μL of each blood and using a Leucocount kit containing beads manufactured by Becton, Dickinson and Company.

$$\text{Residual white blood cell count} = \log\left[\text{leukocyte concentration (cells/}\mu\text{L) (blood after filtration)}\right] \times \text{blood volume (mL)}$$

In the room temperature-stored blood condition, when a residual white blood cell count is less than $1 \times 10^6$ cells (6 Log), the leukocyte removing filter can be considered to be practically desirable. When a residual white blood cell count is less than $1 \times 10^6$ (less than 6 Log/Bag) per bag, severe side effects can be prevented. It is preferably 5.8 Log/Bag or less, more preferably 5.5 Log/Bag or less, and further preferably 5.3 Log/Bag or less. Blood has significant individual differences, and thus the residual white blood cell count (logarithm) is known to show the normal distribution even with the same filter type, approximately with a standard deviation being about 0.20 Log. That is, when the residual white blood cell count per bag is 5.8 Log or less, a safe blood preparation can be made in consideration of variation of 1 σ (65%), when 5.5 Log or less, a safe blood preparation can be made in consideration of variation of 2 σ, and when 5.3 Log or less, a safe blood preparation can be made in consideration of variation of 3 σ. In other words, when the residual white blood cell count per bag is 5.3 Log, a preparation can be made in consideration of a high blood matching ratio of 99.7%, thereby enabling the remarkable reduction of the risk of blood transfusion side effects caused by the residual white blood cell count.

<Capability to recover platelet evaluation>

In addition to the leukocyte removing function, there is a case where the recovery of platelet, which is a useful blood component, is demanded. In this case, it is commonly required to recover 80% or more of the platelets in a blood preparation subjected to the filtration. For this reason, the recovery of platelet, in addition to the residual white blood cell count, is measured as the reference information in the following Examples and Comparative Examples.

Recovery of platelet = (platelet concentration after filtration × volume of blood after filtration) / (platelet concentration before filtration × volume of blood before filtration) × 100 (%)

[Example 1]

(Preparation of filter layer)

Polybutylene terephthalate (PBT) was spun by the melt blown method to form a fiber (fiber base material). Here, PBT resin had an intrinsic viscosity of 0.82 (dL/g), the discharge per single hole was 0.21 (g/min), the hot air pressure at discharge was 0.30 (MPa), and the die temperature at spinning was 280°C.

The fiber after spinning was immersed in a 45% (v/v) ethanol aqueous solution heated to 40°C for 60 minutes, interposed between rubber rolls, and squeezed with a pressure of 0.2 MPa. The same treatment was further carried out again, and the fiber was dried at 60°C for 16 hours.

A copolymer of 2-hydroxyethyl methacrylate (hereinafter, abbreviated as "HEMA") and diethylaminoethyl methacrylate (hereinafter, abbreviated as "DEAEMA") was synthesized by the typical solution radical polymerization. The polymerization reaction was carried out at a monomer concentration in ethanol of 1 mol/L in the presence of 1/200 mol of azoisobutyronitrile (AIBN) as an initiator at 60°C for 8 hours. The generated hydrophilic polymer was dissolved in a 100% ethanol solution thereby to make a coating solution having a concentration of 0.13% (wt/v). The fiber was immersed therein, press-squeezed with rubber rolls to remove an absorbed excess polymer solution, and dried at normal temperature in a draft, thereby to make the fiber whose circumferential surface was coated.

The mass of the coating layer in 1 g of the obtained coating layer-containing fiber was 2.0 mg/g (fiber base material + coating layer). The weight of the fiber per unit area was 92 $g/m^2$, and the specific surface area was 1.25 $m^2/g$.

(Making of blood processing filter)

Sixteen sheets of the coating layer-containing fiber were filled in a soft container having an effective filtration area of 45 $cm^2$ and ultrasonically welded thereby to make a blood processing filter. This blood processing filter was subjected to steam heat treatment at 115°C for 59 minutes and then vacuum-dried at 40°C for 16 hours. In this case, the surface area of the filtration medium was 92 ($g/m^2$) × 45 ($cm^2$)/10000 × 16 (sheets) × 1.25 (m2/g) = 8.3 $m^2$.

ISO 1135-4 Test was carried out using three of this filter and it was found that a maximum absorbance at 240 to 245 nm of the test solution per unit filter was 0.01.

Additionally, the maximum absorbance at 250 to 320 nm was 0.03 and the residual amount on evaporation

was 0.6 mg, both of which satisfied the ISO 1135-4 standards.

Further, a blood filtration test using whole blood was carried out by the method as above and, as a result, the residual white blood cell count was 5.2 Log/Bag, demonstrating good capability to remove leukocyte. For reference, the recovery of platelet was 1%.

[Example 2]

A filter was made by the same method as Example 1, except that the die temperature when spinning the PBT fiber was reduced from 280°C to 260°C and instead an air pressure was increased to 0.4 MPa, and washing treatment by ethanol was not carried out at all. The mass of the coating layer in 1 g of the obtained coating layer-containing fiber was 1.6 mg/g (fiber base material + coating layer). The weight of the fiber was 91 g/$m^2$, and the specific surface area was 1.23 $m^2$/g. The surface area of the filtration medium was 8.2 $m^2$.

ISO 1135-4 Test was carried out using three of this filter and it was found that the maximum absorbance at 240 to 245 nm of the test solution per unit filter was 0.03.

Additionally, the maximum absorbance at 250 to 320 nm was 0.09 and the residual amount on evaporation was 1.2 mg, both of which satisfied the ISO 1135-4 standards. On the other hand, the residual white blood cell count was 5.2 Log/Bag, demonstrating good capability to remove leukocyte. For reference, the recovery of platelet was 1%.

[Example 3]

A filter was made by the same method as Example 1, except that the suction was carried out when spinning the PBT fiber by providing a vent equipment for drawing a vacuum inside the extruder and washing treatment by ethanol was not carried out at all. The mass of the coating layer in 1 g of the obtained coating layer-containing fiber was 1.9 mg/g (fiber base material + coating layer). The weight of the fiber was 92 g/$m^2$, and the specific surface area was 1.2 $m^2$/g. The surface area of the filtration medium was 8.0 $m^2$.

ISO 1135-4 Test was carried out using three of this filter and it was found that the maximum absorbance at 240 to 245 nm of the test solution per unit filter was 0.03.

Additionally, the maximum absorbance at 250 to 320 nm was 0.1 and the residual amount on evaporation was 1.2 mg, both of which satisfied the ISO 1135-4 standards. On the other hand, the residual white blood cell count was 5.3 Log/Bag. For reference, the recovery of platelet was 1%.

[Example 4]

A filter was made by the same method as Example 1, except that the coating treatment by the hydrophilic polymer was not carried out. The weight of the fiber per unit area was 91.8 g/$m^2$, and the specific surface area was 1.26 $m^2$/g.

ISO 1135-4 Test was carried out using three of this filter and it was found that the maximum absorbance at 240 to 245 nm of the test solution per unit filter was 0.005.

Additionally, the maximum absorbance at 250 to 320 nm was 0.01 and the residual amount on evaporation was 0.2 mg, both of which satisfied the ISO 1135-4 standards. On the other hand, the residual white blood cell count was 5.8 Log/Bag. For reference, the recovery of platelet was 0.1%.

[Example 5]

A filter was made by the same method as Example 1, except that a coating solution having a concentration of the hydrophilic polymer of 1.50%(wt/v) was used. The mass of the coating layer in 1 g of the obtained coating layer-containing fiber was 16.0 mg/g (fiber base material + coating layer). The weight of the fiber per unit area was 93.3 g/$m^2$, and the specific surface area was 1.20 $m^2$/g. Due to a large mass of the coating layer, the coating material presumably developed a fine film between fibers and caused a slightly reduced surface area of the fiber.

ISO 1135-4 Test was carried out using three of this filter and it was found that the maximum absorbance at 240 to 245 nm of the test solution per unit filter was 0.03.

Additionally, the maximum absorbance at 250 to 320 nm was 0.08 and the residual amount on evaporation was 4.8 mg, both of which satisfied the ISO 1135-4 standards. On the other hand, the residual white blood cell count was 4.7 Log/Bag. It is specially notable that the present filter had the recovery of platelet of 89%, thereby achieving the compatibility between the leukocyte removal and the platelet recovery.

[Comparative Example 1]

A filter was made by the same method as Example 1, except that the washing treatment was carried out using a 10% (v/v) ethanol aqueous solution. The mass of the coating layer in 1 g of the obtained coating layer-containing fiber was 2.4 mg/g (fiber base material + coating layer). The weight of the fiber per unit area was 92 g/m$^2$, and the specific surface area was 1.25 m$^2$/g.
ISO 1135-4 Test was carried out using three of this filter and it was found that the maximum absorbance at 240 to 245 nm of the test solution per unit filter was 0.07.
Additionally, the maximum absorbance at 250 to 320 nm was 0.15 and the residual amount on evaporation was 0.8 mg, of which the UV value failed to satisfy the ISO 1135-4 standards. On the other hand, the residual white blood cell count was 5.3 Log/Bag. For reference, the recovery of platelet was 1%.

[Comparative Example 2]

A filter was made by the same method as Example 1, except that the washing treatment of the PBT fiber by ethanol was not carried out at all, and 14 sheets of the fiber were used to make the filter. The surface area of the filtration medium was 6.5 m$^2$. The mass of the coating layer in 1 g of the obtained coating layer-containing fiber was 1.5 mg/g (fiber base material + coating layer). The weight of the fiber per unit area was 83 g/m$^2$, and the specific surface area was 1.25 m$^2$/g.
ISO 1135-4 Test was carried out using three of this filter and it was found that the maximum absorbance at 240 to 245 nm of the test solution per unit filter was 0.04.
Additionally, the maximum absorbance at 250 to 320 nm was 0.11 and the residual amount on evaporation was 1.8 mg, of which the UV value failed to satisfy the ISO 1135-4 standards. On the other hand, the residual white blood cell count was 5.9 Log/Bag. For reference, the recovery of platelet was 1%.

[Comparative Example 3]

A filter was made by the same method as Example 1, except that 10 sheets of the fiber were used to make the filter. A surface area of the filtration medium was 5.2 m$^2$.
ISO 1135-4 Test was carried out using three of this filter and it was found that the maximum absorbance at 240 to 245 nm of the test solution per unit filter was 0.007.
Additionally, the maximum absorbance at 250 to 320 nm was 0.02 and the residual amount on evaporation was 0.4 mg, both of which satisfied the ISO 1135-4 standards. On the other hand, the residual white blood cell count was 6.3 Log/Bag, which failed to satisfy the standards. For reference, the recovery of platelet was 2%.

[Comparative Example 4]

The fiber described in Example 2 of Japanese Patent No. 4565762 was made. The washing step, the spinning step under a low-temperature condition, and the vacuum venting step described herein were not carried out. The mass of the coating layer in 1 g of the obtained coating layer-containing fiber was 155.0 mg/g (fiber base material + coating layer). The weight of the fiber was 40 g/m$^2$, and hence a filter was made by the same method as Example 1 using 37 sheets of the fiber so that the fiber weight was equal to the filter of the Example 1. The specific surface area of the fiber was 2.01 m$^2$/g, and accordingly the surface area of the filtration medium was 13.4 m$^2$.
ISO 1135-4 Test was carried out using three of this filter and it was found that the maximum absorbance at 240 to 245 nm of the test solution per unit filter was 0.7.
Additionally, the maximum absorbance at 250 to 320 nm was 1.5 and the residual amount on evaporation was 80 mg, both of which significantly deviated from the ISO 1135-4 standards and hence were unsatisfied. On the other hand, the residual white blood cell count was 4.8 Log/Bag, which satisfied the standards. For reference, the recovery of platelet was 91%.
The reason of failing to satisfy the ISO standards is conceivably that the surface area of the filtration medium is large, and that many fiber-derived components eluted thereby to increase the absorbance. It is also conceived that the mass of the coating layer in 1 g of the coating layer-containing fiber was large, and that thus much of the polymer in the coating layer eluted thereby to increase the residual amount on evaporation.

[Comparative Example 5]

The fiber described in Example 1 of International Publication No. WO2018/034213 was made. The washing step, the spinning step under a low-temperature condition, and the vacuum venting step described herein were not

carried out. The mass of the coating layer in 1 g of the obtained coating layer-containing fiber was 9.0 mg/g (fiber base material + coating layer). The weight of the fiber was 22 g/$m^2$, and hence a filter was made by the same method as Example 1 using 67 sheets of the fiber so that the fiber weight was equal to the filter of Example 1. The specific surface area of the fiber was 1.65 $m^2$/g, and accordingly the surface area of the filtration medium was 10.9 $m^2$.

ISO 1135-4 Test was carried out using three of this filter and it was found that the maximum absorbance at 240 to 245 nm of the test solution per unit filter was 0.55.

Additionally, the maximum absorbance at 250 to 320 nm was 1.2 and the residual amount on evaporation was 8 mg, neither of which satisfied the ISO 1135-4 standards. On the other hand, the residual white blood cell count was 5.1 Log/Bag, which satisfied the standards. For reference, the recovery of platelet was 1%.

[Comparative Example 6]

ISO 1135-4 Test was carried out using PLX-5B-SCD filter for thrombocyte preparations of Asahi Kasei Medical Co., Ltd. and it found that the maximum absorbance at 240 to 245 nm of the test solution per unit filter was 0.05. An unused filter was disassembled and measured, and it was found that the mass of the coating layer in 1 g of the coating layer-containing fiber in the filter was 17.0 mg/g (fiber base material + coating layer). The mass of the filtration medium was 1.26 g, and the surface area was 1.6 $m^2$.

Further, the maximum absorbance at 250 to 320 nm was 0.12, and the residual amount on evaporation was 28 mg, neither of which satisfied the ISO 1135-4 standards. On the other hand, the residual white blood cell count was 7.1 Log/Bag, which failed to satisfy the standards. However, the recovery of platelet as high as 93% was demonstrated. The reason of failing the residual white blood cell count is conceivably because PLX-5B-SCD was the product originally for thrombocyte preparations but used to filter whole blood, which was a different preparation.

[Table 1]

| | | Example 1 | Example 2 | Example 3 | Example 4 | Example 5 |
|---|---|---|---|---|---|---|
| Hot air pressure (MPa) | | 0.3 | 0.4 | 0.3 | 0.3 | 0.3 |
| Die Temperature (°C) | | 280 | 260 | 280 | 280 | 280 |
| Ethanol washing | Concentration (v/v) | 45 | - | - | 45 | 45 |
| | Temperature (°C) | 40 | - | - | 40 | 40 |
| Vacuum venting | | - | - | Performed | - | - |
| Concentration of coating solution (w/v) | | 0.13 | 0.13 | 0.13 | - | 1.5 |
| Surface area of filtration medium ($m^2$)*1 | | 8.3 | 8.2 | 8.0 | 8.3 | 8.1 |
| Pressure loss in ventilation (Pa) | | 1098 | 1052 | 994 | 1103 | 1002 |
| Maximum absorbance (240 to 245 nm)*1 | | 0.01 | 0.03 | 0.03 | 0.005 | 0.03 |
| Residual amount on evaporation (mg)*2 | | 0.6 | 1.2 | 1.2 | 0.2 | 4.8 |
| Maximum absorbance ($\times 10^{-3}/g^{-1}$) (240 to 245nm | | 1.5 | 4.6 | 4.5 | 0.8 | 4.5 |
| Amount of coating (mg/g) | | 2.0 | 1.6 | 1.9 | - | 16.0 |
| Residual white blood cell count (Log/Bag) | | 5.2 (Pass) | 5.2 (Pass) | 5.3 (Pass) | 5.8 (Pass) | 4.7 (Pass) |
| Recovery of platelet (%) | | 1 | 1 | 1 | 0.1 | 89 |
| ISO 1135-4 Elution test [Maximum absorbance (250 to 320 nm)*2] | | Pass [0.03] | Pass [0.09] | Pass [0.11 | Pass [0.01] | Pass [0.08] |
| *1: Value per filter<br>*2: Value per 3 filters | | | | | | |

[Table 2]

| | | Comparative Example 1 | Comparative Example 2 | Comparative Example 3 | Comparative Example 4 | Comparative Example 5 | Comparative Example 6 |
|---|---|---|---|---|---|---|---|
| Hot air pressure (MPa) | | 0.3 | 0.3 | 0.3 | | | |
| Die Temperature (°C) | | 280 | 280 | 280 | | | |
| Ethanol washing | Concentration (v/v) | 10 | - | 45 | | | |
| | Temperature (°C) | 40 | - | 40 | | | |
| Vacuum venting | | - | - | - | | | |
| Concentration of coating solution (w/v) | | 0.13 | 0.13 | 0.13 | | | |
| Surface area of filtration medium ($m^2$)*1 | | 8.3 | 6.5 | 5.2 | 13.4 | 10.9 | 1.6 |
| Maximum absorbance (240 to 245 nm)*1 | | 0.07 | 0.04 | 0.007 | 0.7 | 0.55 | 0.05 |
| Residual amount on evaporation (mg)*2 | | 0.8 | 1.8 | 0.4 | 80 | 8 | 28 |
| Maximum absorbance ($\times 10^{-3}/g^{-1}$) (240 to 245nm) | | 8.5 | 7.6 | 1.4 | 105.1 | 82.9 | 95.6 |
| Amount of coating (mg/g) | | 2.4 | 1.5 | 2.0 | 155.0 | 9.0 | 17.0 |
| Residual white blood cell count (Log/Bag) | | 5.3 (Pass) | 5.9 (Pass) | 6.3 (Fail) | 4.8 (Pass) | 5.1 (Pass) | 7.1 (Fail) |
| Recovery of platelet (%) | | 1 | 1 | 2 | 91 | 1 | 93 |
| ISO 1135-4 Elution test [Maximum absorbance (250 to 320 nm)*2] | | Fail [0.15] | Fail [0.11] | Pass [0.02] | Fail [1.5] | Fail [1.2] | Fail [0.12] |
| *1: Value per filter<br>*2: Value per 3 filters | | | | | | | |

The above results revealed that in the conditions that the aqueous extract of the blood processing filter has a maximum absorbance in the range from 240 to 245 nm of 0.03 or less, and that the surface area of the filter is adjusted to be 6.0 $m^2$ or more, the filter is conformable with the ISO 1135-4 standards while satisfying good leukocyte removing performance.

Further, it is also revealed that the coating layer is suitable as a means to enhance the capability to remove leukocyte. However, since the hydrophilic polymer itself can be an elutable substance, the residual amount on evaporation can be controlled to a lower level by controlling the amount of the coating layer to a certain level or less.

In addition, as shown in Comparative Examples 4 and 6, the increase in the amount of coating was an effective means to increase the recovery of platelet, but in Comparative Examples 4 and 6 the residual amounts on evaporation and the absorbances were also increased. This is presumably because an increased amount of coating enhanced the hydrophilicity of blood thereby to reduce the adsorption of platelet to the fiber. On the other hand, it is conceived that the enhanced immersion property of the fiber base material to the aqueous extract during the ISO test promoted the elution of monomer components and oligomer components in the fiber base material thereby to increase the absorbance. Here, in Example 5, the amount of coating was similarly increased to increase the recovery of platelet, but the reduction in the absorbance was also achieved by carrying out the washing treatment of the nonwoven fabric in advance thereby to decrease the eluted monomer amount and the eluted oligomer amount from the fiber base material.

Industrial Applicability

The blood processing filter according to the present embodiment has the effect of decreasing the residual white blood cell count while being conformable with the ISO standards safety. This brings the benefit to enhance the blood quality in the blood transfusion market and is thus considered to be industrially applicable.

Reference Signs List

1 Container
3 Inlet
4 Outlet
5 Filtration medium
7 Inlet side space
8 Outlet side space
9 Outer-edge part of filtration medium
10 Blood processing filter

**Claims**

1. A blood processing filter comprising:

   a filtration medium including a polyester fiber,
   wherein a surface area of the filtration medium is 6.0 $m^2$ or more, and
   a maximum absorbance of an aqueous extract of the blood processing filter in the range from 240 to 245 nm is 0.03 or less.

2. The blood processing filter according to claim 1, wherein a pressure loss of the blood processing filter in ventilation is 100 to 2000 Pa.

3. The blood processing filter according to claim 1 or 2, wherein a pressure loss of the blood processing filter in ventilation is 400 to 1600 Pa.

4. The blood processing filter according to any one of claims 1 to 3,
   wherein, when 400 mL of a leukocyte-containing liquid having a leukocyte concentration of 5000 cells/$\mu$L is filtered, a leukocyte removing performance is 3.3 or more, wherein the leukocyte removing performance is represented by a formula below:

leukocyte removing performance = −log [(leukocyte concentration in leukocyte-containing liquid after filtration)/(leukocyte concentration in leukocyte-containing liquid before filtration)].

**5.** The blood processing filter according to any one of claims 1 to 3, wherein when 400 mL of a leukocyte-containing liquid having a leukocyte concentration of 5000 cells/μL is filtered, a residual white blood cell count is less than $10^6$ cells.

**6.** The blood processing filter according to any one of claims 1 to 5, wherein the surface area of the filtration medium is 10.0 $m^2$ or less.

**7.** The blood processing filter according to any one of claims 1 to 6, wherein the surface area of the filtration medium is 8.5 $m^2$ or less.

**8.** The blood processing filter according to any one of claims 1 to 7, wherein the maximum absorbance per unit mass (g) of the filtration medium is 0.003 $g^{-1}$ or less.

**9.** The blood processing filter according to any one of claims 1 to 8,

wherein the polyester fiber has a coating layer,
a mass of the coating layer per total mass (g) of the polyester fiber and the coating layer is 15 mg/g or less.

**10.** A method for removing leukocyte, comprising:
the step of passing a leukocyte-containing liquid through the blood processing filter according to any one of claims 1 to 9.

**11.** The method for removing leukocyte according to claim 10, wherein a leukocyte residual rate by the passing is $10^{-3}$ or less, wherein the leukocyte residual rate is represented by a formula below:

leukocyte residual rate = (leukocyte concentration in leukocyte-containing liquid after filtration)/(leukocyte concentration in leukocyte-containing liquid before filtration).

**12.** The method for removing leukocyte according to claim 10, wherein a residual white blood cell count in 400 mL of a leukocyte-containing liquid by the passing is less than $10^6$ cells.

**13.** A method for producing a blood processing filter comprising a filtration medium including a polyester fiber, comprising:

the step of washing the polyester fiber with an ethanol aqueous solution,
wherein a concentration of the ethanol aqueous solution is 12% (v/v) or more, and
a temperature of the ethanol aqueous solution is 20°C or more.

**14.** A method for producing a blood processing filter comprising a filtration medium including a polyester fiber, comprising:
the step of spinning the polyester fiber at 260°C or less.

**15.** A method for producing a blood processing filter comprising a filtration medium including a polyester fiber, comprising:
the step of vacuum venting a polyester source of the polyester fiber.

[Fig. 1]

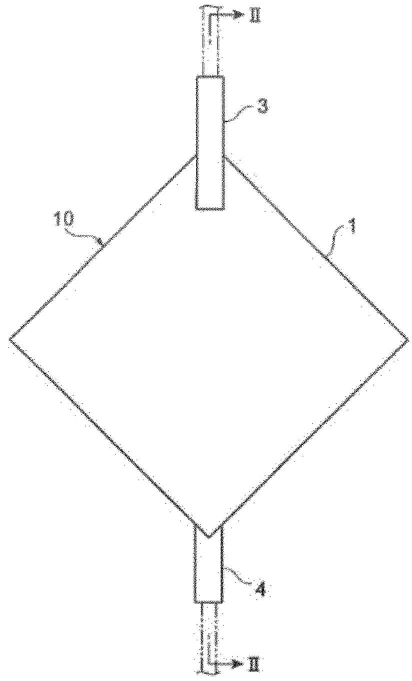


[Fig. 2]

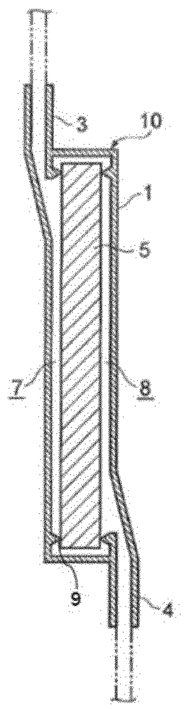

[Fig. 3]

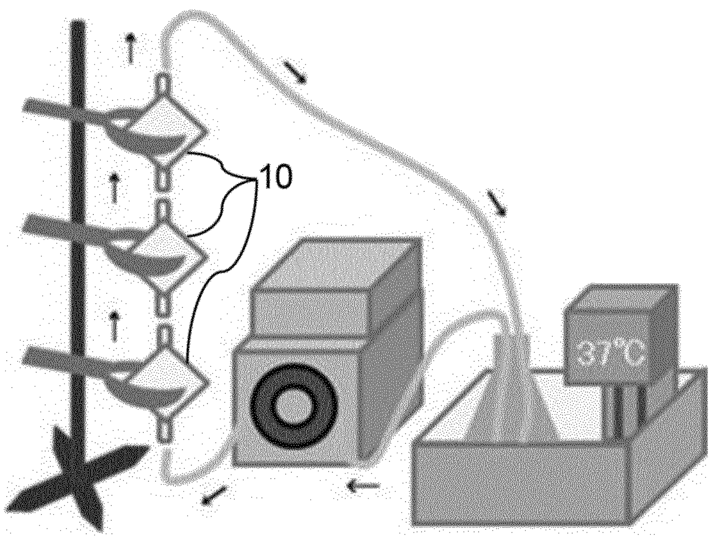
10
37°C

[Fig. 4]

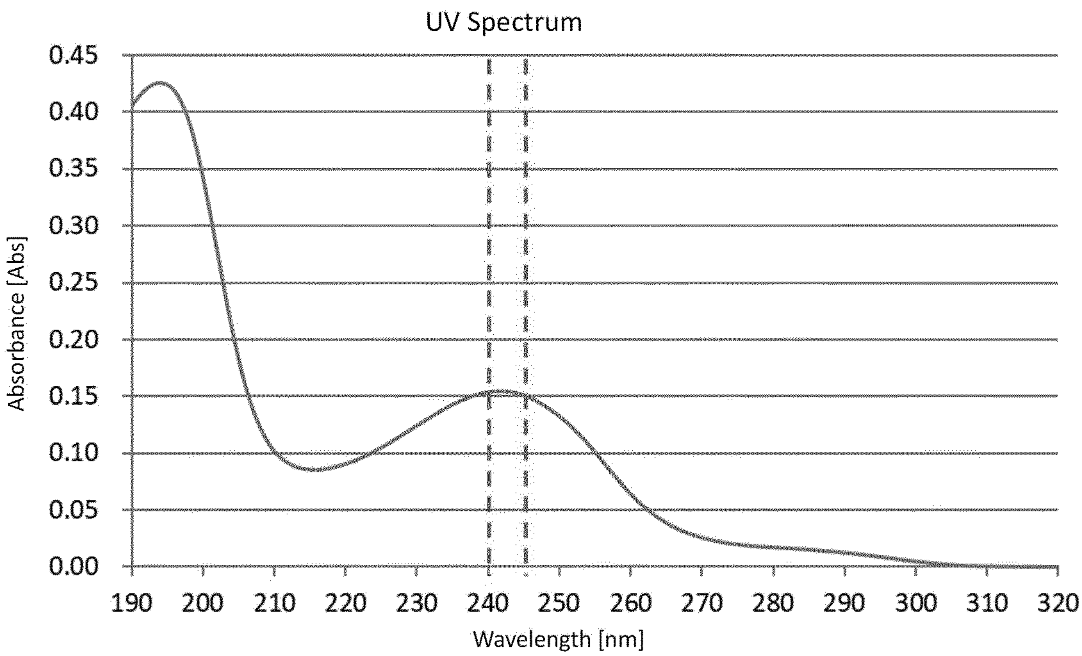
UV Spectrum
0.45
0.40
0.35
0.30
0.25
0.20
0.15
0.10
0.05
0.00
Absorbance [Abs]
190
200
210
220
230
240
250
260
270
280
290
300
310
320
Wavelength [nm]

## INTERNATIONAL SEARCH REPORT

International application No.
PCT/JP2020/038276

A. CLASSIFICATION OF SUBJECT MATTER
B01D 39/16(2006.01)i; A61M 1/02(2006.01)i
FI: A61M1/02 107; B01D39/16 A
According to International Patent Classification (IPC) or to both national classification and IPC

B. FIELDS SEARCHED
Minimum documentation searched (classification system followed by classification symbols)
B01D39/16; A61M1/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2018-507011 A (KOLON INDUSTRIES, INC.) 15 March 2018 (2018-03-15) paragraphs [0071]-[0076] | 14<br>1-13, 15 |
| Y<br>A | WO 01/32236 A1 (ASAHI MEDICAL KK) 10 May 2001 (2001-05-10) specification, page 57, line 20 to page 58, line 11 | 15<br>1-14 |
| Y<br>A | JP 2004-44010 A (TEIJIN LTD.) 12 February 2004 (2004-02-12) paragraphs [0023]-[0024] | 15<br>1-14 |
| A | JP 7-24066 A (ASAHI MEDICAL KK) 27 January 1995 (1995-01-27) paragraphs [0040], [0043] | 1-15 |
| A | WO 03/106518 A1 (ASAHI MEDICAL KK) 24 December 2003 (2003-12-24) specification, page 24, line 16 to page 26, line 12 | 1-15 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 November 2020 (19.11.2020) | 08 December 2020 (08.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/038276

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/196651 A1 (NISSAN CHEMICAL INDUSTRIES, LTD.) 11 December 2014 (2014-12-11) paragraphs [0100]-[0101] | 1-15 |

Form PCT/ISA/210 (continuation of second sheet) (January 2015)

**INTERNATIONAL SEARCH REPORT**
Information on patent family members

International application No.
PCT/JP2020/038276

| Patent Documents referred in the Report | Publication Date | Patent Family | Publication Date |
|---|---|---|---|
| JP 2018-507011 A | 15 Mar. 2018 | US 2017/0354774 A1 paragraphs [0070]-[0076] | |
| WO 01/32236 A1 | 10 May 2001 | EP 1230940 A1 paragraph [0187] | |
| JP 2004-44010 A | 12 Feb. 2004 | (Family: none) | |
| JP 7-24066 A | 27 Jan. 1995 | JP 3386195 B2 | |
| WO 03/106518 A1 | 24 Dec. 2003 | US 2006/0073467 A1 paragraphs [0085]-[0091] | |
| WO 2014/196651 A1 | 11 Dec. 2014 | US 2016/0129176 A1 paragraphs [0113]-[0114] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4565762 B
- WO 2018034213 A
- JP 2006077136 A